# EUROPEAN PATENT SPECIFICATION

(11) **EP 4 389 172 B1**
(45) Date of publication and mention of the grant of the patent: **12.08.2026**
(21) Application number: 22939620.5
(22) Date of filing: 29.06.2022
(51) Int. Cl.: A61M 5/158, A61M 5/142

(54) **INDWELLING SUBCUTANEOUS CATHETER ASSEMBLY**
SUBKUTANE VERWEILKATHETERANORDNUNG
ENSEMBLE CATHÉTER SOUS-CUTANÉ À DEMEURE

(30) Priority: 29.04.2022 CN 202210468463
(43) Date of publication of application: 26.06.2024
(73) Proprietor: Aaruy Medical Electronics Co., Ltd., Shenzhen, Guangdong 518000 (CN)
(72) Inventor: ZHOU, Qingxu, Shenzhen, Guangdong 518000 (CN)
(74) Representative: Petraz, Gilberto Luigi
(86) International application number: PCT/CN2022/102226
(87) International publication number: WO 2023/206792

(56) References cited:
- WO-A1-2020/111080
- CN-A- 101 024 101
- CN-A- 108 926 752
- CN-U- 211 357 119
- CN-U- 212 547 834
- CN-U- 212 547 834
- JP-A- 2006 192 015
- US-A1- 2007 191 772
- US-A1- 2011 046 456

## Description

### Field of the invention

The present application relates to the technical field of medical products, particularly a subcutaneous indwelling needle assembly.

### Background of the invention

Subcutaneous indwelling needles may be left under the human skin for a short period of time, eliminating the demand for frequent puncture operations when infusing medication, thus reducing the pain caused by repeated punctures. The commercially available paste-type medicine delivery devices generally involve the use of subcutaneous indwelling needles, such as insulin infusion pumps commonly used by diabetic patients, which continuously infuse insulin subcutaneously to the patient through the insulin pump by simulating the working mechanism of the human pancreas to ensure that the patient has a normal glucose level. Therefore, the use of subcutaneous indwelling needles is particularly important.

Currently, the paste-type medicine delivery device is generally divided into a separate type and an integrated type. The infusion pump of the separate paste-type medicine delivery device is separated from the subcutaneous indwelling needle and connected through the catheter, which requires the pump to be worn on the belt or related accessories, being inconvenient to use and prone to a series of safety problems such as catheter entanglement and clogging. However, the infusion pump of the existing integrated paste-type medicine delivery device, as well as the subcutaneous indwelling needle are provided on one end of the overall structure. As a human body moves, the whole device is driven up and down like a seesaw, and the indwelling needle is just at the front of the seesaw, which is a position with the largest movement travel in the whole device. With irregular movement, it is difficult to avoid enlargement of the patient's skin wound. US2011/046456A1 discloses an inserter with automatic-release attachment mechanisms that lock to a base during needle insertion and disengage upon needle retraction. US2007/0191772A1 discloses a septum housing that pivots from an upright insertion position to a low-profile latched position post-insertion, minimizing device bulk. CN212547834U focuses on snap-fit assembly between needle/tube bases and retention via sleeve buckles, simplifying component integration.

### Summary of the invention

The objective of the present application is to provide a subcutaneous indwelling needle assembly, so as to improve the reliability of the connection between components in the subcutaneous indwelling needle assembly. The invention is set out in the appended set of claims.

According to an aspect of the present application, provided is a subcutaneous indwelling needle assembly, including a base and a needle assembly; the needle assembly includes a puncture needle holder, a puncture needle, an indwelling needle holder, and a soft indwelling needle; the base includes a baseplate for applying on a surface of skin; the baseplate includes a first surface and a second surface provided oppositely; the base is provided with a perforation hole penetrating the first surface and the second surface; on the first surface, an indwelling needle fixing protrusion for insertion of the indwelling needle holder is formed by protruding along a periphery of the perforation hole; the indwelling needle fixing protrusion is provided with a first restricting part; an exterior wall of the indwelling needle holder is provided with a second restricting part; the first restricting part and the second restricting part, which match each other, are collectively a rotation-stopping mechanism; and the first restricting part and the second restricting part are cooperatively mounted to restrict a relative rotation of the indwelling needle holder with respect to the indwelling needle fixing protrusion when the indwelling needle holder is inserted into the indwelling needle fixing protrusion. On the second surface, a first annular protrusion is formed around a periphery of the perforation hole, and a second annular protrusion is formed around an exterior of the first annular protrusion. When assembling the subcutaneous indwelling needle assembly provided in the present application, the indwelling needle holder is inserted into the indwelling needle fixing protrusion by matching installation between the first restricting part and the second restricting part, which not only ensures a reliable connection between the indwelling needle holder and the base, but also stops a rotation of the indwelling needle holder and the indwelling needle connected to an end of the indwelling needle holder relative to the base. Therefore, after the indwelling needle is inserted into the patient's skin, the indwelling needle is rotated under the patient's skin, which avoids enlargement of the user's skin wound due to irregular movement of the indwelling needle.

Preferably, the first restricting part and the second restricting part are connected by a snap fit.

Preferably, the first restricting part is a first rotation-stopping slot formed by recessing an edge of the indwelling needle fixing protrusion, and the second restricting part is a first rotation-stopping buckle protruding from an exterior wall of the indwelling needle holder.

Preferably, there is a plurality of first restricting parts; the plurality of first restricting parts is evenly distributed at an edge of the indwelling needle fixing protrusion; and the quantity and positional relationship of the second restricting part and the first restricting part correspond to each other. By providing a plurality of evenly distributed first restricting parts and second restricting parts, it is conducive to reinforcing a reliable connection between the indwelling needle holder and the base.

Preferably, an end of the indwelling needle holder proximate to the indwelling needle is provided with a restricting retainment plate; an interior wall of the indwelling needle fixing protrusion is provided with a plurality of elastic restricting retainment buckles; in a direction pointing from the first surface to the second surface, the restricting retainment buckle is tilted in a direction proximate to the indwelling needle; the plurality of restricting retainment buckles are formed as an axial restricting part; a width of an opening of an end of the axial restricting part proximate to the second surface is less than a width of the restricting retainment plate perpendicular to an axis of the indwelling needle; and when the indwelling needle holder is inserted into the indwelling needle fixing platform in a direction pointing from the first surface to the second surface, the restricting retainment plate is protruded from the axial restricting part. In the present solution, when inserting a needle, the indwelling needle holder is inserted into the indwelling needle fixing protrusion in a direction pointing from the first surface to the second surface. The restricting retainment plate of the indwelling needle holder squeezes the restricting retainment buckle so that a free end of the restricting retaining plate moves in a direction distal to the axis of the indwelling needle. The opening of the axial restricting part is thereby stretched open to allow the restricting retainment plate to pass through the axial restricting part. After the restricting retainment plate passes through the axial restricting part, the squeezing effect thereof on the restricting retainment buckle disappears and the restricting retainment buckle is reset, so that the opening of the axial restricting part returns to the size in the natural state. In this case, since a width of the restricting retainment plate is greater than a width of an opening of the axial restricting part, the axial restricting part restricts the restricting retainment plate from passing out of the axial restricting part in a direction pointing from the second surface to the first surface, thereby restricting an axial movement of the indwelling needle holder that is mounted with the base. It may avoid detachment of the indwelling needle holder when the needle is retracted or during use, and it may also restrict movement of the indwelling needle mounted at an end of the indwelling needle holder under the patient's skin.

Preferably, when the indwelling needle holder is inserted into the indwelling needle fixing protrusion, the restricting retainment buckles are abutted against the restricting retainment plate. When the indwelling needle holder is matched and mounted with the base, the indwelling needle holder is clamped into the indwelling needle fixing protrusion of the base by abutting against the restricting retainment plate and the restricting retaining buckle against each other, thereby reinforcing the reliable connection between the indwelling needle holder and the base.

Preferably, an exterior wall of the indwelling needle holder is provided with a third restricting part; the puncture needle holder is provided with a fourth restricting part; the third restricting part and the fourth restricting part, which match each other, are collectively a rotation-stopping mechanism; and the third restricting part and the fourth restricting part are cooperatively mounted to restrict a relative rotation of the indwelling needle holder with respect to the puncture needle holder when the indwelling needle holder is inserted into the puncture needle holder. By matching and mounting the third restricting part and the fourth restricting part, the puncture needle holder stops a rotation relative to the indwelling needle holder, thereby ensuring a reliable connection between the puncture needle holder and the indwelling needle holder, which is conducive to improving the operating speed and the success rate of an operator in inserting an indwelling needle by using a puncture needle connected to one end of the puncture needle holder to pass through the indwelling needle holder.

Preferably, the third restricting part and the fourth restricting part are connected by a snap fit.

Preferably, the third restricting part is a second rotation-stopping buckle protruding from an exterior wall of the indwelling needle holder, and the fourth restricting part is a second rotation-stopping slot formed by recessing an edge of an end of the puncture needle holder proximate to the base.

The second surface of the baseplate is a surface of the baseplate for attaching to the patient's skin. On the second surface of the baseplate, the first annular protrusion and the second annular protrusion mentioned above form a double-ring structure around a periphery of the perforation hole. In attaching to the surface of the patient's skin using the second surface with the double-ring structure mentioned above, the double-ring structure creates a suction effect by squeezing the patient's skin so that the skin enters the gap between the double-ring structure, thereby firmly fixing the subcutaneous indwelling needle assembly to the surface of the patient's skin. In addition, it is also capable of providing a good isolation effect between the infusion area and other skin areas, playing a waterproof role.

Preferably, the perforation hole is provided in a middle of the baseplate. Compared to the case in which the perforation hole is provided at an edge of the baseplate, the perforation hole of the present solution is provided in a middle of the baseplate, which enables the movement of the indwelling needle assembly mounted in the perforation hole relative to the skin to be relatively small, and reduces the damage to the skin caused by a large movement of the indwelling needle assembly during use. Additionally, the design satisfies a balanced design for the installation between the indwelling needle assembly and the baseplate, which may improve the reliability of the connection between the indwelling needle assembly and the baseplate.

Preferably, the baseplate is provided with a plurality of insertion device positioning holes penetrating the first surface and the second surface, the plurality of insertion device positioning holes is evenly distributed on a periphery of the perforation hole. An insertion device for use in conjunction with the subcutaneous indwelling needle assembly provided in the present application includes a cover capable of fitting with the positioning holes. The needle assembly is provided within the cover of the insertion device and is capable of being accurately secured to the center of the baseplate by an ejector mechanism within the insertion device when the cover of the insertion device is fitted with the positioning hole on the baseplate.

Preferably, an upper end of the puncture needle holder is provided with an insertion device rotation-stopping slot, which ensures a reliable connection between the puncture needle and the insertion device.

In the subcutaneous indwelling needle assembly provided in the present application, the base for providing the infusion pump and the indwelling needle assembly is integrated into one unit and attached to the user's abdomen, limbs or other locations without additional connection to a tube, which may be directly attached to the skin for use, omitting a long tube, reducing the volume, and increasing the portability, safety and comfort of wearing the device.

### Brief description of the drawings

Fig. 1 is an exploded diagram of components included in the needle assembly;
Fig. 2 is a structural diagram of the first surface of the base;
Fig. 3 is an enlarged diagram of the indwelling-needle protruding platform;
Fig. 4 is a sectional diagram of the subcutaneous indwelling needle assembly in the assembled state;
Fig. 5 is a structural diagram of the second surface of the base.

In the attached drawings mentioned above, the correspondence of each marking is as follows: 1 puncture needle holder; 11 insertion device rotation-stopping slot; 12 second rotation-stopping slot; 2 puncture needle; 3 indwelling needle holder; 31 second rotation-stopping buckle; 32 first rotation-stopping buckle; 33 restricting retainment plate; 4 indwelling needle; 5 protective cover; 6 insertion device positioning hole; 7 baseplate; 71 first surface; 72 second surface; 73 indwelling needle fixing protrusion; 731 first rotation-stopping slot; 732 restricting retainment buckle; 74 first annular protrusion; 75 second annular protrusion.

### Detailed description of the preferred embodiments

The terms "first", "second", "third", "fourth" and the like in the specification, the claims and the above-mentioned drawings of the present application are used to identify different objects and are not intended to describe a particular sequence or order. It should be understood that the ordinal numbers used in such a way are in appropriate cases interchangeable so that the embodiments described herein may be implemented in an order other than that illustrated or described herein. Additionally, the terms "comprise" and "include" and derivatives thereof are intended to be interpreted non-exclusively. For example, a process, method, system, product or apparatus that includes a series of steps or units is not necessarily limited to those that are clearly listed, but may include other steps or units that are not clearly listed or that are inherent to the process, method, product or apparatus.

### Embodiment 1

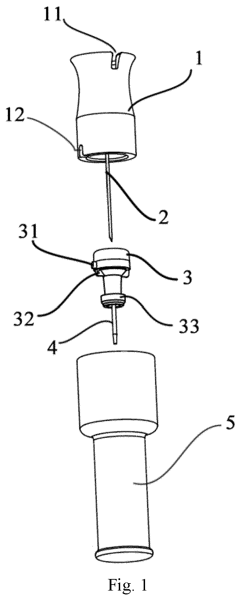

The subcutaneous indwelling needle assembly provided in the present embodiment includes a base and a needle assembly. As shown in Fig. 1, the needle assembly includes a puncture needle holder 1, a puncture needle 2, an indwelling needle holder 3 and a soft indwelling needle 4. An end of the puncture needle holder 1 is fixedly connected to the puncture needle 2, and an end of the indwelling needle holder 3 is fixedly connected to the indwelling needle 4. A bottom exterior wall edge of the puncture needle holder 1 is also provided with a concave second rotation-stopping slot 12. A second rotation-stopping buckle 31 is formed on a top exterior wall of the indwelling needle holder 3 protruding with respect to the exterior wall. A bottom of the puncture needle holder 1 is provided with a groove fitted with a top of the indwelling needle holder 3. When assembling the indwelling needle holder 3 and the puncture needle holder 1, the puncture needle 2 passes through the indwelling needle holder 3 and the indwelling needle 4 sequentially. The second rotation-stopping slot 12 and the second rotation-stopping buckle 31 are snap-fitted with each other, restricting a peripheral rotation of the puncture needle holder 1 with respect to the indwelling needle holder 3. A top of the indwelling needle holder 3 is provided into the groove mentioned above to achieve the abutting of the indwelling needle holder 3 with the puncture needle holder 1, which improves the tightness of the connection between the indwelling needle holder 3 and the puncture needle holder 1, thereby achieving a reliable connection between the puncture needle holder 1 and the indwelling needle holder 3. According to the demand of practical usage, a protective cover 5 may also be provided to match the needle assembly mentioned above. Before assembling the needle assembly and the base, the protective cover 5 is used to sleeve on an exterior of the needle assembly to protect the needle assembly.

In some implementations, an insertion device rotation-stopping slot 11 may also be provided at an upper end of the puncture needle holder 1 for an insertion device matched for use therewith, thereby ensuring a reliable connection between the puncture needle 2 and the insertion device.

In particular, an exterior wall of the indwelling needle holder 3 mentioned above is also provided with a first rotation-stopping buckle 32 protruding with respect to the exterior wall thereof, the first rotation-stopping buckle 32 being provided below the second rotation-stopping buckle 31.

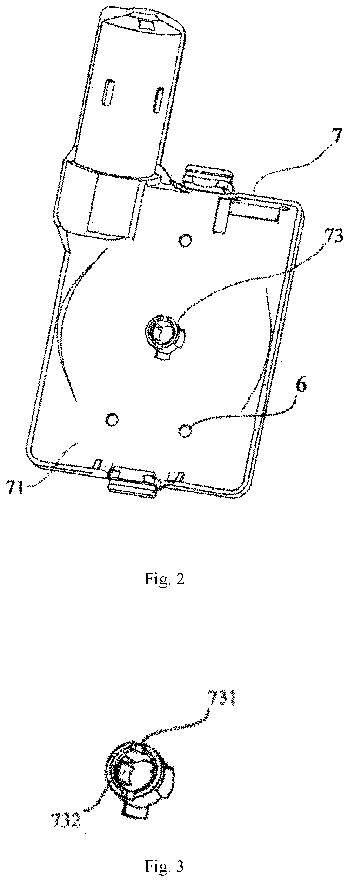

As shown in Fig. 2, Fig. 3 and Fig. 5, the base includes a baseplate 7; the baseplate 7 includes a first surface 71 and a second surface 72 provided oppositely; the base is provided with a perforation hole penetrating the first surface 71 and the second surface 72; and on the first surface 71, an indwelling needle fixing protrusion 73 for insertion of the indwelling needle holder 3 is formed by protruding along a periphery of the perforation hole. In the present embodiment, an edge of the indwelling needle fixing protrusion 73 is recessed to form a first rotation-stopping slot 731 that matches the first rotation-stopping buckle 32 mentioned above; the dimensions and the distributing position of the first rotation-stopping slot 731 and the first rotation-stopping buckle 32 match each other. In the present embodiment, the first rotation-stopping slot 731 and the first rotation-stopping buckle 32 are formed in one-to-one correspondence. Two first rotation-stopping slots 731 symmetrically distributed with respect to a central axis of the indwelling needle fixing protrusion 73 are provided on an edge of the indwelling needle fixing protrusion 73. Two first rotation-stopping buckles 32 symmetrically distributed with respect to a central axis of the indwelling needle holder 3 are provided on an exterior wall of the indwelling needle holder 3. When the indwelling needle holder 3 is inserted into the indwelling needle fixing protrusion 73, each of the first rotation-stopping buckles 32 snaps into the first rotation-stopping slot 731 correspondingly provided therewith, thereby restricting the peripheral rotation of the indwelling needle holder 3 with respect to the indwelling needle fixing protrusion 73, and thereby achieving the positioning of the indwelling needle 4 connected to the indwelling needle holder 3 on the base, and avoiding enlargement of the user skin wound area due to irregular movement of the indwelling needle 4 during use.

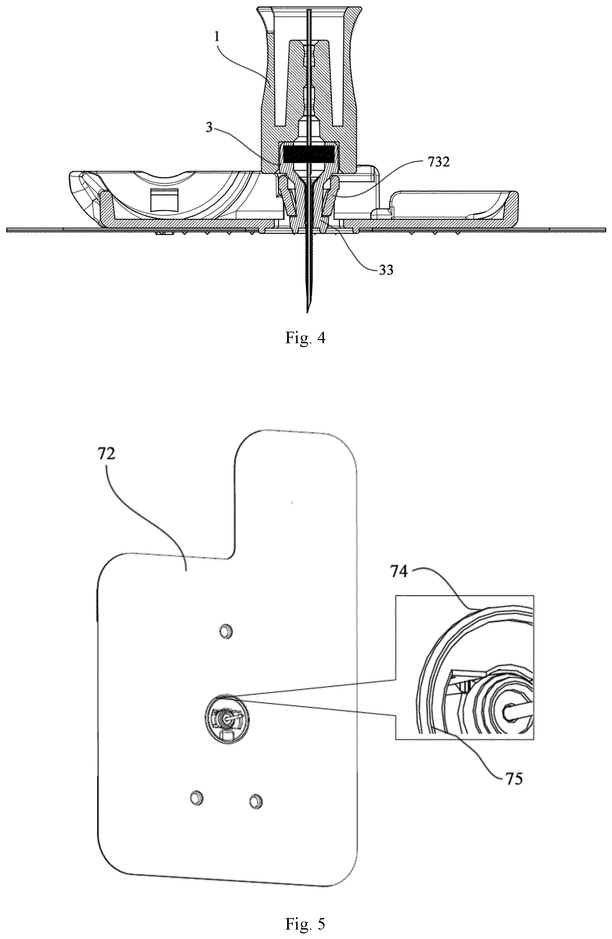

Further, as shown in Fig. 4, an interior wall of the indwelling needle fixing protrusion 73 is also provided with an elastic restricting retainment buckle 732. In a direction pointing from the first surface 71 to the second surface 72, the restricting retainment buckle 732 is tilted in a direction proximal to the indwelling needle 4. In the present embodiment, the amount of the restricting retainment buckles 732 is two; two restricting retainment buckles 732 are symmetrically provided with respect to the center axis of the indwelling needle fixing protrusion 73; and the axial restricting part is formed by two restricting retainment buckles 732 mentioned above. A bottom of the indwelling needle holder 3 is also provided with a restricting retainment plate 33, which in the present embodiment is a rounded platform connected to the indwelling needle 4. A diameter of the restricting retainment plate 33 is greater than the width of an opening at an end of the axial restricting part proximate to the second surface 72. When inserting a needle, the indwelling needle holder 3 is inserted into the indwelling needle fixing protrusion 73 in a direction pointing from the first surface 71 to the second surface 72. The restricting retainment plate 33 of the indwelling needle holder 3 squeezes the restricting retainment buckle 732 so that a free end of the restricting retainment plate 33 moves in a direction distal to an axis of the indwelling needle 4. An opening of the axial restricting part is thereby stretched open to allow the restricting retainment plate 33 to pass through the axial restricting part. After the restricting retainment plate 33 passes through the axial restricting part, the squeezing effect thereof on the restricting retainment buckle 732 is gone and the restricting retainment buckle 732 is reset, so that an opening of the axial restricting part returns to the size in the natural state. In this case, since a width of the restricting retainment plate 33 is greater than a width of an opening of the axial restricting part, the axial restricting part restricts the restricting retainment plate 33 from passing out of the axial restricting part in a direction pointing from the second surface 72 to the first surface 71, thereby restricting an axial movement of the indwelling needle holder 3 that is mounted with the base. It may avoid detachment of the indwelling needle holder 3 when the needle is retracted or during use, and it may also restrict movement of the indwelling needle 4 mounted at an end of the indwelling needle holder 3 under the patient's skin. In the present embodiment, when the indwelling needle holder 3 is inserted into the indwelling needle fixing protrusion 73, the restricting retainment buckles 732 are abutted against the restricting retainment plate 33. Therefore, when the indwelling needle holder 3 is matched and mounted with the base, the indwelling needle holder 3 is clamped into the indwelling needle fixing protrusion 73 of the base by abutting against the restricting retainment plate 33 and the restricting retainment buckle 732 against each other, thereby reinforcing the reliable connection between the indwelling needle holder 3 and the base.

Additionally, as shown in Fig. 5, on the second surface 72 of the baseplate 7 adopted by the present embodiment, a first annular protrusion 74 is formed around a periphery of the perforation hole, and a second annular protrusion 75 is formed around an exterior of the first annular protrusion 74. The first annular protrusion 74, the second annular protrusion 75 and the perforation hole are all provided on a same axis. The second surface 72 of the baseplate 7 is a surface of the baseplate 7 for attaching to the patient's skin. On the second surface 72 of the baseplate 7, the first annular protrusion 74 and the second annular protrusion 75 mentioned above form a double-ring structure around a periphery of the perforation hole. In attaching to a surface of the patient's skin using the second surface 72 with the double-ring structure mentioned above, the double-ring structure creates a suction effect by squeezing the patient's skin so that the skin enters a gap between the double-ring structure, thereby firmly fixing the subcutaneous indwelling needle assembly to a surface of the patient's skin. In addition, it is also capable of providing a good isolation effect between the infusion area and other skin areas, playing a waterproof role.

In the present embodiment, the perforation hole is provided in a middle of the baseplate 7. The baseplate 7 is provided with a plurality of insertion device positioning holes 6 penetrating the first surface 71 and the second surface 72, the plurality of insertion device positioning holes 6 are evenly distributed on a periphery of the perforation hole. Compared to the case in which the perforation hole is provided at an edge of the baseplate 7, the perforation hole of the present solution is provided in a middle of the baseplate 7, which enables the movement of the indwelling needle assembly mounted in the perforation hole relative to the skin to be relatively small, and reduces the damage to the skin caused by a large movement of the indwelling needle 4 assembly during use. Additionally, the design satisfies a balanced design for the installation between the indwelling needle 4 assembly and the baseplate 7, which may improve the reliability of the connection between the indwelling needle 4 assembly and the baseplate 7. An insertion device for use in conjunction with the subcutaneous indwelling needle assembly provided in the present application includes a cover capable of fitting with the positioning holes. The needle assembly is provided within the cover of the insertion device and is capable of being accurately secured to a center of the baseplate 7 by an ejector mechanism within the insertion device when the cover of the insertion device is fitted with the insertion device positioning hole 6 on the baseplate 7. Depending on the demand of practical usage, an adhesive tape for attaching to the patient's skin may also be attached to the second surface 72, and the adhesive tape may be attached to the baseplate 7 by ultrasonic welding or adhesive fixation.

The above examples are only used to illustrate the technical solution of the present application rather than to limit the protection scope of the present application. Although the present application has been described in detail with reference to the above examples, a person of ordinary skill in the art should be aware that modifications or substitutions may be carried out to the technical solution of the present application, these modifications or substitutions fall within the protection scope of the present application.

## Claims

1. A subcutaneous indwelling needle assembly, comprising a base and a needle assembly;
the needle assembly comprises a puncture needle holder (1), a puncture needle (2), an indwelling needle holder (3), and a soft indwelling needle (4);
the base comprises a baseplate (7) for applying on a surface of skin; the baseplate (7) comprises a first surface (71) and a second surface (72) provided oppositely; the base is provided with a perforation hole penetrating the first surface (71) and the second surface (72); on the first surface (71), an indwelling needle fixing protrusion (73) for insertion of the indwelling needle holder (3) is formed by protruding along a periphery of the perforation hole;
the indwelling needle fixing protrusion (73) is provided with a first restricting part; an exterior wall of the indwelling needle holder (3) is provided with a second restricting part; the first restricting part and the second restricting part, which match each other, are collectively a rotation-stopping mechanism; and the first restricting part and the second restricting part are cooperatively mounted to restrict a relative rotation of the indwelling needle holder (3) with respect to the indwelling needle fixing protrusion (73) when the indwelling needle holder (3) is inserted into the indwelling needle fixing protrusion (73);
**characterized in that**
on the second surface (72), a first annular protrusion (74) is formed around a periphery of the perforation hole, and a second annular protrusion (75) is formed around an exterior of the first annular protrusion (74).

2. The subcutaneous indwelling needle assembly according to claim 1, wherein the first restricting part and the second restricting part are connected by a snap fit.

3. The subcutaneous indwelling needle assembly according to claim 2, wherein the first restricting part is a first rotation-stopping slot (731) formed by recessing an edge of the indwelling needle fixing protrusion (73), and the second restricting part is a first rotation-stopping buckle (32) protruding from an exterior wall of the indwelling needle holder (3).

4. The subcutaneous indwelling needle assembly according to claim 3, wherein there is a plurality of first restricting parts; the plurality of first restricting parts is evenly distributed at an edge of the indwelling needle fixing protrusion (73); and quantity and positional relationship of the second restricting part and the first restricting part correspond to each other.

5. The subcutaneous indwelling needle assembly according to claim 1, wherein
an end of the indwelling needle holder (3) proximate to the indwelling needle (4) is provided with a restricting retainment plate (33); an inner wall of the indwelling needle fixing protrusion (73) is provided with a plurality of elastic restricting retainment buckles (732); in a direction pointing from the first surface (71) to the second surface (72), the restricting retainment buckle (732) is tilted in a direction proximate to the indwelling needle (4);
the plurality of restricting retainment buckles (732) are formed as an axial restricting part; a width of an opening of an end of the axial restricting part proximate to the second surface (72) is less than a width of the restricting retainment plate (33) perpendicular to an axis of the indwelling needle (4); and when the indwelling needle holder (3) is inserted into the indwelling needle fixing protrusion (73) in a direction pointing from the first surface (71) to the second surface (72), the restricting retainment plate (33) is protruded from the axial restricting part.

6. The subcutaneous indwelling needle assembly according to claim 5, wherein when the indwelling needle holder (3) is inserted into the indwelling needle fixing protrusion (73), the restricting retainment buckles (732) are abutted against the restricting retainment plate (33).

7. The subcutaneous indwelling needle assembly according to claim 1, wherein an exterior wall of the indwelling needle holder (3) is provided with a third restricting part; the puncture needle holder (1) is provided with a fourth restricting part; the third restricting part and the fourth restricting part, which match each other, are collectively a rotation-stopping mechanism; and the third restricting part and the fourth restricting part are cooperatively mounted to restrict a relative rotation of the indwelling needle holder (3) with respect to the puncture needle holder (1) when the indwelling needle holder (3) is inserted into the puncture needle holder (1).

8. The subcutaneous indwelling needle assembly according to claim 7, wherein the third restricting part and the fourth restricting part are connected by a snap fit.

9. The subcutaneous indwelling needle assembly according to claim 7, wherein the third restricting part is a second rotation-stopping buckle (31) protruding from an exterior wall of the indwelling needle holder (3), and the fourth restricting part is a second rotation-stopping slot (12) formed by recessing an edge of an end of the puncture needle holder (1) proximate to the base.

10. The subcutaneous indwelling needle assembly according to claim 1, wherein the perforation hole is provided in a middle of the baseplate (7).

11. The subcutaneous indwelling needle assembly according to claim 10, wherein the baseplate (7) is provided with a plurality of insertion device positioning holes (6) penetrating the first surface (71) and the second surface (72), the plurality of insertion device positioning holes (6) is evenly distributed on a periphery of the perforation hole.

## Patentansprüche

1. Subkutane Verweilnadelanordnung, umfassend eine Basis und eine Nadelanordnung;
die Nadelanordnung umfasst einen Punktionsnadelhalter (1), eine Punktionsnadel (2), einen Verweilnadelhalter (3) und eine weiche Verweilnadel (4);
die Basis umfasst eine Basisplatte (7) zur Aufbringung auf einer Hautfläche; die Basisplatte (7) umfasst eine erste Fläche (71) und eine zweite Fläche (72), die entgegengesetzt vorgesehen sind; die Basis ist mit einem Perforationsloch versehen, das die erste Fläche (71) und die zweite Fläche (72) durchdringt; an der ersten Fläche (71) ist ein Verweilnadelbefestigungsvorsprung (73) für die Einfügung des Verweilnadelhalters (3) durch Vorspringen entlang eines Umfangs des Perforationsloches ausgebildet;
der Verweilnadelbefestigungsvorsprung (73) ist mit einem ersten einschränkenden Teil versehen; eine Außenwand des Verweilnadelhalters (3) ist mit einem zweiten einschränkenden Teil versehen; der erste einschränkende Teil und der zweite einschränkende Teil, die zueinander passen, sind gemeinsam ein Drehungsstoppmechanismus; und der erste einschränkende Teil und der zweite einschränkende Teil sind zusammenwirkend montiert, um eine relative Drehung des Verweilnadelhalters (3) mit Bezug auf den Verweilnadelbefestigungsvorsprung (73) einzuschränken, wenn der Verweilnadelhalter (3) in den Verweilnadelbefestigungsvorsprung (73) eingefügt ist;
**dadurch gekennzeichnet, dass**
an der zweiten Fläche (72), ein erster ringförmiger Vorsprung (74) um einen Umfang des Perforationsloches ausgebildet ist, und ein zweiter ringförmiger Vorsprung (75) um einen Außenbereich des ersten ringförmigen Vorsprungs (74) ausgebildet ist.

2. Subkutane Verweilnadelanordnung nach Anspruch 1, worin der erste einschränkende Teil und der zweite einschränkende Teil durch eine Schnapppassung verbunden sind.

3. Subkutane Verweilnadelanordnung nach Anspruch 2, worin der erste einschränkende Teil ein erster Drehungsstoppschlitz (731) ist, der durch Vertiefen eines Randes des Verweilnadelbefestigungsvorsprungs (73) ausgebildet ist, und der zweite einschränkende Teil eine erste Drehungsstoppwölbung (32) ist, die aus einer Außenwand des Verweilnadelhalters (3) herausragt.

4. Subkutane Verweilnadelanordnung nach Anspruch 3, worin es eine Vielzahl von ersten einschränkenden Teilen gibt; die Vielzahl von ersten einschränkenden Teilen an einem Rand des Verweilnadelbefestigungsvorsprungs (73) gleichmäßig verteilt ist; und Menge und Lagebeziehung des zweiten einschränkenden Teils und des ersten einschränkenden Teils einander entsprechen.

5. Subkutane Verweilnadelanordnung nach Anspruch 1, worin
ein Ende des Verweilnadelhalters (3), das der Verweilnadel (4) nahe ist, mit einer einschränkenden Halteplatte (33) versehen ist; eine Innenwand des Verweilnadelbefestigungsvorsprungs (73) mit einer Vielzahl von elastischen einschränkenden Haltewölbungen (732) versehen ist; in einer Richtung, die von der ersten Fläche (71) zur zweiten Fläche (72) zeigt, die einschränkende Haltewölbung (732) in einer Richtung geneigt ist, die nahe der Verweilnadel (4) ist;
die Vielzahl von einschränkenden Haltewölbungen (732) als ein axialer einschränkender Teil ausgestaltet ist; eine Breite einer Öffnung eines Endes des axialen einschränkenden Teils, das der zweiten Fläche (72) nahe ist, kleiner ist als eine Breite der einschränkenden Halteplatte (33), die zu einer Achse der Verweilnadel (4) senkrecht ist; und wenn der Verweilnadelhalter (3) in den Verweilnadelbefestigungsvorsprung (73) in einer Richtung eingefügt ist, die von der ersten Fläche (71) zur zweiten Fläche (72) zeigt, die einschränkende Halteplatte (33) aus dem axialen einschränkenden Teil herausragt.

6. Subkutane Verweilnadelanordnung nach Anspruch 5, worin, wenn der Verweilnadelhalter (3) in den Verweilnadelbefestigungsvorsprung (73) eingefügt ist, die einschränkenden Haltewölbungen (732) gegen die einschränkende Halteplatte (33) gestoßen werden.

7. Subkutane Verweilnadelanordnung nach Anspruch 1, worin eine Außenwand des Verweilnadelhalters (3) mit einem dritten einschränkenden Teil versehen ist; der Punktionsnadelhalter (1) mit einem vierten einschränkenden Teil versehen ist; der dritte einschränkende Teil und der vierte einschränkende Teil, die zueinander passen, gemeinsam ein Drehungsstoppmechanismus sind; und der dritte einschränkende Teil und der vierte einschränkende Teil zusammenwirkend montiert sind, um eine relative Drehung des Verweilnadelhalters (3) mit Bezug auf den Punktionsnadelhalter (1) einzuschränken, wenn der Verweilnadelhalter (3) in den Punktionsnadelhalter (1) eingefügt ist.

8. Subkutane Verweilnadelanordnung nach Anspruch 7, worin der dritte einschränkende Teil und der vierte einschränkende Teil durch eine Schnapppassung verbunden sind.

9. Subkutane Verweilnadelanordnung nach Anspruch 7, worin der dritte einschränkende Teil eine zweite Drehungsstoppwölbung (31) ist, die aus einer Außenwand des Verweilnadelhalters (3) herausragt, und der vierte einschränkende Teil ein zweiter Drehungsstoppschlitz (12) ist, der durch Vertiefen eines Randes eines Endes des Punktionsnadelhalters (1), das der Basis nahe ist, ausgebildet ist.

10. Subkutane Verweilnadelanordnung nach Anspruch 1, worin das Perforationsloch in einer Mitte der Basisplatte (7) vorgesehen ist.

11. Subkutane Verweilnadelanordnung nach Anspruch 10, worin die Basisplatte (7) mit einer Vielzahl von Einfügungseinrichtungs-Positionierlöchern (6) versehen ist, die die erste Fläche (71) und die zweite Fläche (72) durchdringen, die Vielzahl von Einfügungseinrichtungs-Positionierlöchern (6) an einem Umfang des Perforationsloches gleichmäßig verteilt ist.

## Revendications

1. Ensemble aiguille sous-cutanée à demeure, comprenant une base et un ensemble aiguille ;
l'ensemble aiguille comprend un porte-aiguille de ponction (1), une aiguille de ponction (2), un porte-aiguille à demeure (3), et une aiguille à demeure souple (4) ;
la base comprend une plaque de base (7) pour appliquer sur une surface de la peau ; la plaque de base (7) comprend une première surface (71) et une seconde surface (72) prévues à l'opposé ; la base est pourvue d'un trou de perforation pénétrant dans la première surface (71) et la seconde surface (72) ; sur la première surface (71), une saillie de fixation d'aiguille à demeure (73) pour l'insertion du porte-aiguille à demeure (3) est formée en faisant saillie le long d'une périphérie du trou de perforation ;
la saillie de fixation d'aiguille à demeure (73) est pourvue d'une première partie de restriction ; une paroi extérieure du porte-aiguille à demeure (3) est pourvue d'une deuxième partie de restriction ; la première partie de restriction et la deuxième partie de restriction, qui se correspondent, sont collectivement un mécanisme d'arrêt de rotation ; et la première partie de restriction et la deuxième partie de restriction sont montées de manière coopérative pour limiter une rotation relative du porte-aiguille à demeure (3) par rapport à la saillie de fixation d'aiguille à demeure (73) lorsque le porte-aiguille à demeure (3) est inséré dans la saillie de fixation d'aiguille à demeure (73) ;
**caractérisé en ce que** sur la seconde surface (72), une première saillie annulaire (74) est formée autour d'une périphérie du trou de perforation, et une seconde saillie annulaire (75) est formée autour d'un extérieur de la première saillie annulaire (74).

2. Ensemble aiguille sous-cutanée à demeure selon la revendication 1, dans lequel la première partie de restriction et la deuxième partie de restriction sont reliées par un ajustement par encliquetage.

3. Ensemble aiguille sous-cutanée à demeure selon la revendication 2, dans lequel la première partie de restriction est une première fente d'arrêt de rotation (731) formée en évidant un bord de la saillie de fixation d'aiguille à demeure (73), et la deuxième partie de restriction est une première boucle d'arrêt de rotation (32) faisant saillie d'une paroi extérieure du porte-aiguille à demeure (3).

4. Ensemble aiguille sous-cutanée à demeure selon la revendication 3, dans lequel il y a une pluralité de premières parties de restriction ; la pluralité de premières parties de restriction est répartie uniformément au niveau d'un bord de la saillie de fixation d'aiguille à demeure (73) ; et la quantité et la relation de position de la deuxième partie de restriction et de la première partie de restriction correspondent l'une à l'autre.

5. Ensemble aiguille sous-cutanée à demeure selon la revendication 1, dans lequel
une extrémité du porte-aiguille à demeure (3) à proximité de l'aiguille à demeure (4) est pourvue d'une plaque de retenue de restriction (33) ; une paroi intérieure de la saillie de fixation d'aiguille à demeure (73) est pourvue d'une pluralité de boucles de retenue de restriction élastiques (732) ; dans une direction pointant de la première surface (71) à la seconde surface (72), la boucle de retenue de restriction (732) est inclinée dans une direction à proximité de l'aiguille à demeure (4) ;
la pluralité de boucles de retenue de restriction (732) sont formées comme une partie de restriction axiale ; une largeur d'une ouverture d'une extrémité de la partie de restriction axiale à proximité de la seconde surface (72) est inférieure à une largeur de la plaque de retenue de restriction (33) perpendiculaire à un axe de l'aiguille à demeure (4) ; et lorsque le porte-aiguille à demeure (3) est inséré dans la saillie de fixation d'aiguille à demeure (73) dans une direction pointant de la première surface (71) à la seconde surface (72), la plaque de retenue de restriction (33) fait saillie de la partie de restriction axiale.

6. Ensemble aiguille sous-cutanée à demeure selon la revendication 5, dans lequel lorsque le porte-aiguille à demeure (3) est inséré dans la saillie de fixation d'aiguille à demeure (73), les boucles de retenue de restriction (732) sont en butée contre la plaque de retenue de restriction (33).

7. Ensemble aiguille à demeure sous-cutanée selon la revendication 1, dans lequel une paroi extérieure du porte-aiguille à demeure (3) est pourvue d'une troisième partie de restriction ; le porte-aiguille de ponction (1) est pourvu d'une quatrième partie de restriction ; la troisième partie de restriction et la quatrième partie de restriction, qui se correspondent, sont collectivement un mécanisme d'arrêt de rotation ; et la troisième partie de restriction et la quatrième partie de restriction sont montées en coopération pour limiter une rotation relative du porte-aiguille à demeure (3) par rapport au porte-aiguille de ponction (1) lorsque le porte-aiguille à demeure (3) est inséré dans le porte-aiguille de ponction (1).

8. Ensemble aiguille sous-cutanée à demeure selon la revendication 7, dans lequel la troisième partie de restriction et la quatrième partie de restriction sont reliées par un ajustement par encliquetage.

9. Ensemble aiguille sous-cutanée à demeure selon la revendication 7, dans lequel la troisième partie de restriction est une seconde boucle d'arrêt de rotation (31) faisant saillie à partir d'une paroi extérieure du porte-aiguille à demeure (3), et la quatrième partie de restriction est une seconde fente d'arrêt de rotation (12) formée en évidant un bord d'une extrémité du porte-aiguille de ponction (1) à proximité de la base.

10. Ensemble aiguille sous-cutanée à demeure selon la revendication 1, dans lequel le trou de perforation est prévu au milieu de la plaque de base (7).

11. Ensemble aiguille sous-cutanée à demeure selon la revendication 10, dans lequel la plaque de base (7) est pourvue d'une pluralité de trous de positionnement de dispositif d'insertion (6) pénétrant dans la première surface (71) et la seconde surface (72), la pluralité de trous de positionnement de dispositif d'insertion (6) est répartie uniformément sur une périphérie du trou de perforation.
